(19) Europäisches Patentamt
European Patent Office
Office européen des brevets

(11) Publication number: **0 008 905**
**B1**

(12) **EUROPEAN PATENT SPECIFICATION**

(45) Date of publication of patent specification: **16.02.83**

(21) Application number: **79301703.9**

(22) Date of filing: **21.08.79**

(51) Int. Cl.$^3$: **A 61 K 31/42,**
A 61 K 31/43,
A 61 K 31/545 //(A61K31/43, 31/42), (A61K31/545, 31/42)

(54) **Pharmaceutical compositions containing two beta-lactam derivatives.**

(30) Priority: **06.09.78 GB 3570878**

(43) Date of publication of application:
**19.03.80 Bulletin 80/6**

(45) Publication of the grant of the patent:
**16.02.83 Bulletin 83/7**

(84) Designated Contracting States:
**BE CH DE FR GB IT NL SE**

(56) References cited:
**EP - A - 0 000 526**
**DE - A - 2 559 411**
**DE - A - 2 608 079**
**DE - A - 2 810 056**
**FR - A - 2 374 031**
**GB - A - 2 005 538**

**The file contains technical information submitted after the application was filed and not included in this specification**

(73) Proprietor: **BEECHAM GROUP PLC**
**Beecham House Great West Road**
**Brentford Middlesex (GB)**

(72) Inventor: **Taskis, Charles Bernard**
**Hillside, Durrington Hill**
**Worthing, Sussex (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 008 905 B1**

Courier Press, Leamington Spa, England

Pharmaceutical compositions containing two beta-lactam derivatives

This invention relates to pharmaceutical compositions. More specifically this invention relates to anti-bacterial pharmaceutical compositions which may be administered by injection.

U.K. Patent No. 1508977 describes clavulanic acid, of formula (I):

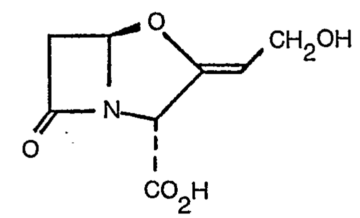

(I)

and its salts; and the use of such compounds as synergists for penicillin and cephalosporins.

The administration of such synergistic formulations by injection is also described, the injectable compositions being made up by the addition of water to the synergistic formulations.

It has been found that certain of these injectable compositions lose potency fairly quickly on reconstitution with water so that they have to be administered shortly after this reconstitution.

It is an object of this invention to provide an injectable pharmaceutical composition having improved stability relative to the hereinbefore described compositions.

This object is attained by the inclusion of a specific polyvinylpyrrolidone in the compositions.

Accordingly the present invention provides a pharmaceutical composition, which on reconstitution with water yields an injectable solution, which composition comprises a water soluble salt of clavulanic acid, a water soluble salt of an $\alpha$-amino penicillin or cephalosporin and polyvinylpyrrolidone of molecular weight 1000 to 12000.

The polyvinylpyrrolidone (hereafter referred to as PVP) used in this invention will have a molecular weight of from 1000 to 12000. Since the material is a polymer it will be realised by a chemist that the molecular weight referred to is an average molecular weight. A suitable method of determining the average molecular weight of PVP for use in this invention is gel permeation chromatography. The PVP should not contain molecules with a molecular weight of more than 30,000. Similarly it should not have a monomer content of more than 1%. The K value of suitable PVP will generally be between 10 and 18.

Favourably the PVP employed will have a molecular weight of 1500 to 6000.

A preferred PVP for use in the invention will have a molecular weight of 2000—3500. A PVP of this kind is Kollidon CE 5080 (Kollidon is a Registered Trade Mark) which is available from BASF Aktiengesellschaft, D-6700 Ludwigshafen, Federal Republic of Germany. This PVP has a K value of 12 to 14 which is a favoured range.

Normally the weight ratio of active ingredients (taken as the free acid equivalent weight) to PVP in the compositions of the invention will be 1:0.25 to 1:5, more suitably 1:0.5 to 1:3.

The ratio of clavulanate salt to penicillin or cephalosporin salt present in the composition will normally be in the range 10:1 to 1:10, usually in the range 1:5 to 1:1, and will preferably be 1:2 [These ratios are weight/weight ratios based on free acid equivalent weights].

Suitably the clavulanate salt will be the sodium or potassium salt, preferably the postassium salt.

Suitably the penicillin or cephalosporin salt is the sodium or potassium salt, preferably the sodium salt.

$\alpha$-Amino penicillins and cephalosporins have a side chain of structure (II):

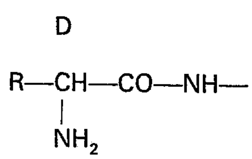

(II)

(joined at the 6- or 7- position respectively).

A particularly useful class of such penicillins and cephalosporins has R in structure (II) being phenyl, $p$-hydroxyphenyl or cyclohexadienyl. Examples of such penicillins include ampicillin and amoxycillin, and of such cephalosporins include cephradine..

It has been found that this invention is particularly pertinent to formulations wherein the penicillin is amoxycillin.

Thus one preferred embodiment of this invention is a pharmaceutical composition comprising sodium or potassium clavulanate, sodium or potassium amoxycillin, and PVP.

In this preferred embodiment the relative proportions of the ingredients are suitably as hereinbefore described.

Most preferably in this embodiment the active ingredients are potassium clavulanate and sodium amoxycillin.

It has been found that the compositions of this invention can also usefully contain a water soluble solid acidic material. Suitable examples of such materials include organic acids such as citric, tartaric, malic, ascorbic, gluconic, maleic, succinic and aconitic acids. Preferably such acids will not contain a hydroxy group. It is presently believed that maleic acid is one of the most preferred organic acids to include in the compositions of the invention. Suitable acidic materials also include inorganic materials such as sodium dihydrogen phosphate.

When the aforesaid acidic materials are present in the compositions, then suitably the weight ratio of active ingredients (as free acid) to acidic material is in the range 100:1 to 10:1, more suitably 75:1 to 25:1.

Generally we have found that, when an acidic material is present, higher proportions of the acid can be used when higher proportions of PVP are present.

The compositions of this invention may be reconstituted with an aqueous solvent, such as water, in conventional manner, the ingredients either being dissolved simultaneously or consecutively.

It will be appreciated that as the compositions of this invention are to be used only after reconstitution into a solution, then the physical form of the ingredients of the compositions, and (when appropriate) the homogeneity of mixtures thereof, is not of fundamental importance to the invention. Thus while conveniently powdered ingredients and (when appropriate) intimate powder mixtures are used, we have found that the product of the process described in our copending Patent Application (corresponding to European Patent Application Publication No. 0012496) of even date entitled "A process for the preparation of sodium amoxycillin" claiming priority from U.K. Patent Application No. 47744/78, filed 8th December, 1978, (the disclosure of which is hereby incorporated by reference), may also be used. This product is a powder comprising a solid solution of a water soluble salt of amoxycillin in the PVP.

The compositions of this invention will normally be presented in a glass vial. Such vials may be filled in conventional manner with the desired active ingredient and PVP in powder form. However conveniently as a first step a solution of the clavulanate salt is freeze dried *in situ* in the vial and the other ingredients added to the resultant plug.

It will be appreciated that for the sake of ease of reconstitution the composition is preferably presented in vials, each of which vials contain a unit dose of the composition.

The compositions of this invention may be used in the manner described in U.K. Patent No. 1508977.

In a further aspect, the invention provides a method of improving the stability of an aqueous injectable solution of a water soluble salt of clavulanic acid and a water soluble salt of an $\alpha$-amino penicillin or cephalosporin, which method comprises incorporating in the solution PVP of molecular weight 1000 to 12000.

The following Examples illustrate the invention.

### Example 1

The following composition was prepared by mixing together the stated ingredients in the stated proportions:

| | |
|---|---|
| 250 mg p.f.a. | Sodium Amoxycillin |
| 125 mg p.f.a. | Potassium Clavulanate |
| 15 mg | d-Tartaric Acid |
| 700 mg | PVP (Kollidon CE 5080) |

This composition was reconstituted with 2 ml of water.

### Example 2

Example 1 was repeated exactly, but using 17.5 mg of maleic acid in place of the 20 mg of d-tartaric acid.

### Example 3

Reconstituted compositions were prepared containing 250 mg p.f.a. of sodium amoxycillin and 125 mg p.f.a. of potassium clavulanate in 2 ml of water. The amounts of acid and PVP (Kollidon CE 5080) used in these compositions was as shown in the Table 1:

TABLE 1

| Example Number | Acid | Weight mg | P.V.P. Content % w/v |
|---|---|---|---|
| 3a | Aspartic | 30 | 40 |
| 3b | Glutamic | 22.5 | 50 |
| 3c | Glutaric | 17.5 | 35 |
| 3d | Citric | 20 | 50 |
| 3e | Maleic | 17.5 | 35 |
| 3f | Malic | 20 | 35 |
| 3g | Succinic | 22.5 | 50 |
| 3h | Tartaric | 22.5 | 50 |

Example 4

Reconstituted compositions were prepared containing 500 mg p.f.a. of sodium amoxycillin and 250 mg p.f.a. of potassium clavulanate in 4 ml of water. The amounts of acid and PVP (Kollidone CE 5080) used in these compositions was as shown in Tables 2, 3 and 4.

TABLE 2

| | | | |
|---|---|---|---|
| Citric Acid mg. | 17.5 | 22.5 | 20.0 |
| P.V.P. % w/v | 40 | 55 | 50 |

TABLE 3

| | A | B | C | D | E |
|---|---|---|---|---|---|
| Tartaric Acid mg. | 20 | 22.5 | 20 | 17.5 | 15 |
| P.V.P. % w/v | 40 | 35 | 35 | 35 | 35 |

TABLE 4

| | A | B | C | D |
|---|---|---|---|---|
| Maleic Acid mg. | 20 | 15 | 17.5 | 17.5 |
| P.V.P. % w/v | 35 | 30 | 35 | 30 |

Example 5

Example 1 was repeated exactly, with the exception that the 20 mg. of d-tartaric acid was omitted.

Example 6

The following try mix was prepared:

| | |
|---|---|
| Sodium Amoxycillin | 250 mg p.f.a. |
| Potassium Clavulanate | 125 mg p.f.a. |
| PVP (Kollidone 12 PF) | 600 mg |
| $NaH_2PO_4$ | 37.5 mg |

This composition was reconstituted in water (3 ml) to give a solution of pH 8.3. This solution incurred a 10% potency loss of clavulanic acid after 15 minutes.

**Claims**

1. A pharmaceutical composition, which on reconstitution with water yields an injectable solution, which composition comprises a water soluble salt of clavulanic acid, a water soluble salt of an $\alpha$-amino penicillin or cephalosporin, and polyvinylpyrrolidone (PVP) of molecular weight 1000 to 12000.

2. A composition according to claim 1, wherein the PVP has a molecular weight of 2000 to 3500.

3. A composition according to claim 1 or claim 2, wherein the weight ratio of active ingredients (taken as the free acid equivalent weight) to PVP is 1:0.5 to 1:3.

4. A composition according to any one of the preceding claims, wherein the weight ratio of clavulanate salt to penicillin or cephalosporin salt (weights taken as the free acid equivalent weight) is 1:5 to 1:1.

5. A composition according to any one of the preceding claims wherein the clavulanate salt is sodium or potassium.

6. A composition according to any one of the preceding claims, wherein the pencillin is sodium or potassium ampicillin, or sodium or potassium amoxycillin; or the cephalosporin is sodium or potassium cephradine.

7. A composition according to any one of the preceding claims, wherein the active ingredients are potassium clavulanate and sodium amoxycillin.

8. A composition according to any one of the preceding claims, also containing a water soluble acidic material.

9. A composition according to claim 8 wherein the water soluble acidic material is citric acid, tartaric acid, malic acid, ascorbic acid, gluconic acid, maleic acid, succinic acid or aconitic acid.

10. A composition according to any one of the preceding claims, when reconstituted for injection with water.

11. A vial, which vial contains a unit dose of the composition according to any one of the claims 1 to 9.

12. A method for improving the stability of an aqueous injectable solution of a water soluble salt of clavulanic acid and a water soluble salt of an $\alpha$-aminopenicillin or cephalosporin, which method comprises incorporating in the solution PVP of molecular weight 1000 to 12000.

**Patentansprüche**

1. Eine pharmazeutische Zusammensetzung, die bei der Rekonstitution mit Wasser eine injizierbare Lösung gibt, wobei die Zusammensetzung ein wasserlösliches Salz der Clavulansäure, ein wasserlösliches Salz eines $\alpha$-Aminopenicillins oder Cephalosporins und Polyvinylpyrrolidon (PVP) mit einem Molekulargewicht von 1000 bis 12000 enthält.

2. Eine Zusammensetzung gemäß Anspruch 1, wobei das PVP ein Molekulargewicht von 2000 bis 3500 hat.

3. Eine Zusammensetzung gemäß Anspruch 1 oder Anspruch 2, wobei das Gewichtsverhältnis von Wirkstoffen (berechnet als Äquivalentgewicht der freien Säure) zu PVP 1:0,5 bis 1:3 ist.

4. Eine Zusammensetzung gemäß einem der vorstehenden Ansprüche, wobei das Gewichtsverhältnis von Clavulansäure-Salz zu Penicillin- oder Cephalosporin-salz (Gewichte als Äquivalentgewichte der freien Säure berechnet) 1:5 bis 1:1 ist.

5. Eine Zusammensetzung gemäß einem der vorstehenden Ansprüche, wobei das Clavulansäure-Salz ein Natrium- oder Kaliumsalz ist.

6. Eine Zusammensetzung gemäß einem der vorstehenden Ansprüche, wobei das Penicillin Natrium- oder Kalium-Ampicillin oder Natrium- oder Kalium-Amoxycillin oder das Cephalosporin Natrium- oder Kalium-Cephradin ist.

7. Eine Zusammensetzung gemäß einem der vorstehenden Ansprüche, wobei die Wirkstoffe Kaliumclavulanat und Natrium-Amoxycillin sind.

8. Eine Zusammensetzung gemäß einem der vorstehenden Ansprüche, die auch ein wasserlösliches saures Material enthält.

9. Eine Zusammensetzung gemäß Anspruch 8, wobei das wasserlösliche saure Material Zitronensäure, Weinsäure, Apfelsäure, Ascorbinsäure, Gluconsäure, Maleinsäure, Bernsteinsäure oder Aconitsäure ist.

10. Eine Zusammensetzung gemäß einem der vorstehenden Ansprüche, die mit Wasser zu Injektionen rekonstituiert worden ist.

11. Eine Ampulle, wobei die Ampulle eine Einzeldosis der Zusammensetzung gemäß einem der Ansprüche 1 bis 9 enthält.

12. Eine Methode zur Verbesserung der Stabilität einer wäßrigen injizierbaren Lösung eines wasserlöslichen Salzes der Clavulansäure und eines wasserlöslichen Salzes eines $\alpha$-Aminopenicillins oder Cephalosporins, wobei die Methode die Maßnahme umfaßt, daß in die Lösung PVP mit einem Molekulargewicht von 1000 bis 12000 einverleibt wird.

**Revendications**

1. Composition pharmaceutique qui, par reconstitution avec de l'eau, donne une solution injectable, cette composition refermant un sel soluble dans l'eau d'acide clavulanique, un sel soluble dans l'eau d'une $\alpha$-aminopénicilline ou céphalosporine et de la polyvinylpyrrolidone (PVP) d'un poids moléculaire de 1000 à 12000.

2. Composition suivant la revendication 1, caractérisée en ce que la PVP a un poids moléculaire de 2000 à 3500.

3. Composition suivant la revendication 1 ou 2, caractérisée en ce que le rapport en poids entre les substances actives (considérées comme poids équivalent d'acide libre) et la PVP est de 1:0.5 à 1:3.

4. Composition suivant l'une quelconque des revendications précédentes, caractérisée en ce que le rapport en poids entre la clavulanate et le sel de pénicilline ou de céphalosporine (poids considéré comme poids équivalent d'acide libre) est compris entre 1:5 et 1:1.

5. Composition suivant l'une quelconque des revendications précédentes, caractérisée en ce que le clavulanate est le sel de sodium ou de potassium.

6. Composition suivant l'une quelconque des revendications précédentes, caractérisée en ce que la pénicilline est l'ampicilline sodique ou potassique ou l'amoxicilline sodique ou potassique, ou bien la céphalosporine est la céphradine sodique ou potassique.

7. Composition suivant l'une quelconque des revendications précédentes, caractérisée en ce que les substances actives sont le clavulanate de potassium et l'amoxicilline sodique.

8. Composition suivant l'une quelconque des revendications précédentes, contenant également une matière acide soluble dans l'eau.

9. Composition suivant la revendication 8, caractérisée en ce que la matière acide soluble dans l'eau est de l'acide citrique, de l'acide tartrique, de l'acide malique, de l'acide ascorbique, de l'acide gluconique, de l'acide maléique, de l'acide succinique ou de l'acide aconitique.

10. Composition suivant l'une quelconque des revendications précédentes, caractérisée en ce qu'elle est reconstituée pour l'injection avec de l'eau.

11. Flacon renfermant une dose unitaire de la composition suivant l'une quelconque des revendications 1 à 9.

12. Procédé pour améliorer la stabilité d'une solution aqueuse injectable d'une sel soluble dans l'eau d'acide clavulanique et d'un sel soluble dans l'eau d'une $\alpha$-amino-pénicilline ou céphalosporine, ce procédé consistant à incorporer à la solution de la PVP d'un poids moléculaire de 1000 à 12000.